(19) Europäisches Patentamt
European Patent Office
Office européen des brevets

(11) EP 3 943 544 A1

(12) **EUROPEAN PATENT APPLICATION**
published in accordance with Art. 153(4) EPC

(43) Date of publication:
**26.01.2022 Bulletin 2022/04**

(21) Application number: **20762217.6**

(22) Date of filing: **28.02.2020**

(51) International Patent Classification (IPC):
***C08L 5/16*** (2006.01) ***C08L 89/00*** (2006.01)
***A61K 49/08*** (2006.01) ***A61K 49/12*** (2006.01)
***A61K 49/14*** (2006.01) ***G01N 24/00*** (2006.01)

(52) Cooperative Patent Classification (CPC):
**A61K 49/08; A61K 49/12; A61K 49/14; C08L 5/16; C08L 89/00; G01N 24/00;** Y02A 90/30

(86) International application number:
**PCT/JP2020/008481**

(87) International publication number:
**WO 2020/175695 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2019 JP 2019036868**

(71) Applicants:
- **Kyushu University, National University Corporation**
  **Nishi-ku**
  **Fukuoka-shi**
  **Fukuoka 819-0395 (JP)**
- **RIKEN**
  **Wako-shi**
  **Saitama 351-0198 (JP)**

(72) Inventors:
- **YANAI, Nobuhiro**
  **Fukuoka-shi, Fukuoka 819-0395 (JP)**
- **KIMIZUKA, Nobuo**
  **Fukuoka-shi, Fukuoka 819-0395 (JP)**
- **KAWASHIMA, Yusuke**
  **Fukuoka-shi, Fukuoka 819-0395 (JP)**
- **NISHIMURA, Koki**
  **Fukuoka-shi, Fukuoka 819-0395 (JP)**
- **KOUNO, Hironori**
  **Fukuoka-shi, Fukuoka 819-0395 (JP)**
- **FUJIWARA, Saiya**
  **Fukuoka-shi, Fukuoka 819-0395 (JP)**
- **TATEISHI, Kenichiro**
  **Saitama 351-0198 (JP)**
- **UESAKA, Tomohiro**
  **Saitama 351-0198 (JP)**

(74) Representative: **Zimmermann & Partner Patentanwälte mbB**
**Postfach 330 920**
**80069 München (DE)**

(54) **COMPOSITION AND COMPOSITE**

(57) Disclosed is a composition of a polarization source dispersed in an aqueous medium. Preferably, the polarization source forms a composite with a host. As the aqueous medium, a hydrogel is also usable in addition to water.

EP 3 943 544 A1

## Description

### Technical Field

The present invention relates to a composition and a composite useful in hypernuclear polarization.

### Background Art

When an atomic nucleus (nuclear spin) having a magnetic moment is put in a magnetostatic field, it precesses, and in that condition, when it is irradiated with an electromagnetic wave having the same frequency as that of the precessional motion, the nuclear spin resonates to provide a nuclear magnetic resonance (NMR) phenomenon to absorb the energy of the electromagnetic field. The resonant frequency in the NMR phenomenon differs depending on the nuclear species and the chemical or magnetic environment where the atomic nucleus is left, and therefore in the field of organic chemistry and biochemistry, the frequency spectrum (chemical shift value) of the NMR signal of an electric signal converted from the amount of energy absorption by the resonance is observed to analyze the molecular structure and the physical properties of a compound, which is referred to as NMR spectroscopy much employed in the art. On the other hand, in the field of medicine, a magnetic resonance imaging method (MRI) where positional information is given to the NMR signal to make a picture is employed for non-invasive examination of biological organs such as brain.

When a magnetostatic field is applied to an aggregate of nuclear spins, and for example, in the case of protons, the spins cleave into an energy state facing parallel to the magnetic field and an energy state facing antiparallel to the magnetic field. Here, a value calculated by dividing the difference between the numbers of the spins (spin population) each in a different energy state by the total number of the spins is referred to as a polarization ratio, and the intensity of an NMR signal is said to be proportional to the polarization ratio. However, the polarization ratio of nuclear spins is generally a fraction of several tens of thousands at room temperature and is an extremely small value, and this is one reason of limiting the sensitivity in NMR spectroscopy and MRI.

Given the situation, as a method for hyperpolarization (hypernuclear polarization) of nuclear spins, there is proposed a dynamic nuclear polarization method of hyperpolarizing nuclear spins by transitioning spin polarization of electrons to ambient nuclei by a solid effect or an integrated solid effect induced by electromagnetic wave irradiation. Here, as a supply source for electron spins, a radical and a photoexcited triplet molecule are used, and among these, a photoexcited triplet molecule takes such a state that the electron spin population is significantly biased to a specific energy state irrespective of temperature, and therefore even at room temperature, the nuclear spins can be effectively hyperpolarized as one advantage.

Regarding a dynamic nuclear polarization system using such a photoexcited triplet molecule, NPL 1 describes an organic crystal substrate of p-terphenyl doped with pentacene as a photoexcited triplet molecule, and NPL 2 proposes an amorphous substrate of o-terphenyl doped with pentacene as a photoexcited triplet molecule.

### Citation List

### Non-Patent Literature

NPL 1: PNAS, 2014, 111, 7529
NPL 2: Angew. Chem. Int. Ed., 2013, 52, 13307

### Summary of Invention

### Technical Problem

As mentioned above, NPL 1 proposes a dynamic nuclear polarization system of an organic crystal substrate of p-terphenyl doped with pentacene as a photoexcited triplet molecule, and NPL 2 proposes a dynamic nuclear polarization system of an amorphous substrate of o-terphenyl doped with pentacene as a photoexcited triplet molecule. However, conventional hypernuclear polarization is limited to use for nuclei in a solid crystal, and is not suitable at all for use for living bodies and bio-related materials (e.g., body compositions and analogues of body compositions).

Given the situation, for the purpose of solving such problems of the related arts, the present inventors have assiduously made extensive studies to realize hypernuclear polarization in a medium capable of paving the way for application to living bodies.

Solution to Problem

For solving the above-mentioned problems, the present inventors have made assiduous studies, paying specific attention to an aqueous medium as a medium for hypernuclear polarization, and as a result, have found that even in an aqueous medium, a function of a polarization source can be expressed. With that, the inventors have reached an idea that such a system makes it possible to apply hypernuclear polarization to living bodies and bio-related materials. The present invention is proposed based on such a finding, and specifically has the following constitution.

[1] A composition of a polarization source dispersed in an aqueous medium.
[2] The composition according to [1], wherein the polarization source is dispersed in an aqueous medium in a non-associated state therein.
[3] The composition according to [1] or [2], wherein a composite of the polarization source and a host is dispersed in an aqueous medium.
[4] The composition according to [3], wherein the composite is particles having a particle size of 800 nm or less.
[5] The composition according to [3] or [4], wherein the composite contains a surfactant.
[6] The composition according to [3] or [4], wherein the host is a protein.
[7] The composition according to [6], wherein the host is an albumin.
[8] The composition according to [3] or [4], wherein the host is an oligosaccharide.
[9] The composition according to [8], wherein the oligosaccharide is a cyclodextrin.
[10] The composition according to any one of [1] to [9], wherein the aqueous medium is water.
[11] The composition according to any one of [1] to [9], wherein the aqueous medium is a hydrogel.
[12] The composition according to [11], wherein the polarization source forms a composite with the hydrogel.
[13] The composition according to [11], wherein the polarization source is incorporated in the hydrogel by covalent bonding.
[14] A composite of a polarization source dispersed in a host and having a particle size of 800 nm or less.
[15] The composite according to [14], having a surfactant.
[16] The composite according to [14] or [15], wherein the host is a protein.
[17] The composite according to [16], wherein the host is an albumin.
[18] The composite according to [14] or [15], wherein the host is an oligosaccharide.
[19] The composite according to [18], wherein the oligosaccharide is a cyclodextrin.

Advantageous Effects of Invention

The composition of the present invention enables hypernuclear polarization in an aqueous medium. The composition of the present invention uses an aqueous medium, and is therefore readily applicable to living bodies and bio-related materials.

Brief Description of Drawings

[Fig. 1] This is a schematic view for explaining a dynamic nuclear polarization mechanism of the composition of the present invention.
[Fig. 2] This is an SEM picture of pentacene-doped nanoparticles produced in Example 1.
[Fig. 3] This is a particle size distribution of pentacene-doped nanoparticles produced in Example 1.
[Fig. 4] This shows $^{1}H$ NMR spectra after dynamic nuclear polarization for a predetermined period of time of an aqueous dispersion containing pentacene-doped nanoparticles.
[Fig. 5] This shows $^{1}H$ NMR spectra after dynamic nuclear polarization for 60 seconds of an aqueous dispersion containing pentacene-doped nanoparticles.
[Fig. 6] This shows $^{1}H$ spin polarization buildup curves in dynamic nuclear polarization of an aqueous dispersion containing pentacene-doped nanoparticles and pentacene-doped bulk crystals.
[Fig. 7] This is a reaction scheme used in producing a composite-containing gel 2.
[Fig. 8] This shows a temporal change of an ESR spectrum peak intensity of composite-containing gels 1 and 2, a composite-containing aqueous dispersion 3 and a comparative gel 1.
[Fig. 9] This is a $^{1}H$ NMR spectrum after dynamic nuclear polarization for a predetermined period of time of a composite-containing gel 1.
[Fig. 10] This is a graph showing irradiation time-dependence of an NMR integrated value of a composite-containing

gel 1.

Description of Embodiments

Hereinunder the contents of the present invention are described in detail. The description of the constitutive elements given hereinunder is for some typical embodiments and specific examples of the invention, but the invention should not be limited to such embodiments and specific examples. In this description, the numerical range expressed by the wording "a number to another number" means the range that falls between the former number indicating the lower limit of the range and the latter number indicating the upper limit thereof. The hydrogen atom that is present in the molecule of a compound used in the invention is not particularly limited in isotope species, and for example, all the hydrogen atoms in the molecule may be $^1H$, and all or a part of them may be $^2H$ (deuterium (D)). In this description, "excitation light" is a light to excite a target object to cause radiation or emission, and can be a light having the same wavelength as the absorption wavelength of the target object.

<Composition>

The composition of the present invention contains a polarization source dispersed in an aqueous medium.

In the composition of the present invention, a polarization source exists in an aqueous medium as dispersed therein, and therefore, the polarization source can fully express the function thereof to transition the spin polarization of the formed electron to nuclei to increase the spin polarization of the nuclei. In addition, the composition of the present invention uses an aqueous medium, and can be readily applied to living bodies and bio-related materials.

Hereinunder the components that the composition of the present invention contain are described.

[Polarization Source]

The "polarization source" in the present invention is a supply source of spin polarization that forms a polarized electron spin to supply the electron spin polarization to nuclei.

The polarization source is not specifically limited so far as it can form a polarized electron spin, but is preferably a molecule capable of taking an excited triplet state, since the molecule of the type can form an electron spin having a high polarization ratio irrespective of temperature. An excited triplet state has multiple sub-levels differing in the magnetic quantum number m, and can form a high spin polarization state of which the electron spin population is greatly biased to a specific sub-level. Consequently, using a molecule capable of taking an excited triplet state as a polarization source, the nuclear spin polarization ratio can be effectively increased.

Here, "molecule capable of taking an excited triplet state" means a molecule capable of transitioning to an excited triplet state when given an excitation energy applied thereto. Transition into an excited triplet state may be transition from a ground singlet state to an excited triplet state to directly occur by application of an excitation energy to the molecule, or may also be intersystem crossing from an excited singlet state formed by application of an excitation energy to an excited triplet state. The excitation energy to cause transition into an excited state may be an energy of an excitation light, or may be a recombination energy of an injected carrier, or may be an excitation energy received from a different molecule having been in an excited state.

The molecule capable of taking an excited triplet state may be an inorganic molecule or an organic molecule, but is preferably an organic molecule.

A preferred organic molecule usable as a polarization source is a compound having a skeleton of 4 to 6 condensed benzene rings. Specific examples of the skeleton having 4 to 6 condensed benzene rings include a tetracene skeleton, a pentacene skeleton, a hexacene skeleton, a rubrene skeleton, and a picene skeleton, and may also include structures of two or more kinds of these skeletons bonding to each other, as well as structures of these skeletons with a benzene ring, a naphthalene ring or a biphenyl ring bonding thereto. The compound having a skeleton of 4 to 6 condensed benzene rings may be an unsubstituted compound composed of the skeleton alone, or may be a derivative having a structure of these skeletons substituted with a substituent, but is preferably a derivative. Regarding preferred ranges and specific examples of substituents substitutable on the skeleton, reference may be made to the preferred ranges and the specific examples of the substituents that R to be mentioned below may have. Above all, the substituent of the skeleton preferably contains a functional group that interacts with the host and the hydrogel to be mentioned hereinunder. With that, the molecule of polarization source can be dispersed to a high degree with ease. Such a functional group includes an acid group such as a carboxy group (-COOH), a sulfo group ($-SO_3H$), a phosphono group ($-P(O)(OH)_2$) and a phosphonoxy group ($-OP(O)(OH)_2$), and an anionic group derived from these groups by dissociation of protons therefrom. A carboxy group and a carboxylate anion group are preferred.

Also preferably, at least a part of the hydrogen atoms in the compound to be used as a polarization source are substituted with deuterium atoms, and more preferably, 30 to 70% of the hydrogen atoms existing in the molecule are

substituted with deuterium atoms. With that, the spin-lattice relaxation time of the polarization source can be prolonged and the nuclear spin can be effectively hyperpolarized. Here, the site of the compound to be substituted with a deuterium atom is preferably a relatively movable site. For example, in the case where a skeleton of 4 to 6 condensed benzene rings to constitute the compound has an atomic group (substituent) bonding thereto via a single bond, preferably, at least a part of the hydrogen atoms that the substituent has are substituted with deuterium atoms, more preferably all the hydrogen atoms are substituted with deuterium atoms. A preferred example of the substituent to be substituted with deuterium atoms is an alkyl group having 1 to 20 carbon atoms.

The compound to be used as a polarization source is preferably a compound represented by the following general formula (A) or a salt thereof.

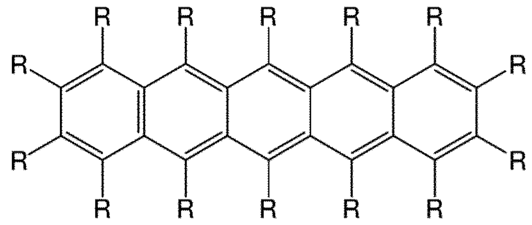

(A)

In the formula (A), R each independently represents a hydrogen atom (H), a deuterium atom (D), or a hydrocarbon group having 1 to 20 carbon atoms and optionally having at least one atom selected from the group consisting of an oxygen atom, a sulfur atom and a silicon atom.

In the formula (A), R each independently represents a hydrogen atom (H), a deuterium atom (D), or a hydrocarbon group having 1 to 20 carbon atoms and optionally having at least one atom selected from the group consisting of an oxygen atom, a sulfur atom and a silicon atom. The "hydrocarbon group" is not limited to a linear saturated hydrocarbon group but may have a carbon-carbon unsaturated bond, a branched structure and a cyclic structure. The wording "optionally having at least one atom selected from the group consisting of an oxygen atom, a sulfur atom and a silicon atom" means that the group may include a functional group containing an oxygen atom, a sulfur atom or a silicon atom, and may also include a linking group containing an oxygen atom, a sulfur atom or a silicon atom inside the carbon skeleton or at the terminal thereof. Accordingly, the hydrocarbon group "optionally having at least one atom selected from the group consisting of an oxygen atom, a sulfur atom and a silicon atom" includes, for example, a hydrocarbon group having 2 carbon atoms and containing a hydroxy group, such as $-CH_2-CH_2-OH$, a hydrocarbon group having 2 carbon atoms containing an ether group inside the carbon skeleton, such as $-CH_2-O-CH_3$, and a hydrocarbon group having 2 carbon atoms and containing an ether group at the terminal of the carbon skeleton, such as $-O-CH_2-CH_3$.

"A salt thereof" of a pentacene derivative is a compound of a pentacene derivative having an acid center such as a carboxy group (-COOH) or a sulfo group ($-SO_3H$), and the salt thereof means that the hydrogen ion in the compound is replaced with a metal cation.

In the case where R is a hydrocarbon group, the carbon number of the hydrocarbon group is preferably 3 or more, more preferably 6 or more, and is preferably 12 or less, more preferably 8 or less.

The hydrocarbon group includes a phenyl group, a biphenyl group, a phenylthio group, a decylthio group and an ethynyl group.

The functional group and the linking group that are contained in the hydrocarbon group include a carboxyl group (-COOH), a potassium salt of a carboxy group (-COOK), a sulfo group ($-SO_3H$), a thioether group (-S-), a triethylsilyl group ($-SiEt_3$) and a triisopropylsilyl group ($-SiiPr_3$).

The bonding number of the hydrocarbon groups is generally 1 or more, preferably 2 or more, and is generally 6 or less, preferably 5 or less.

The bonding position of the hydrocarbon groups includes a combination of a 6-position and a 13-position (the bonding number of the hydrocarbon groups is 2), a combination of a 5-position, a 7-position, a 12-position and a 14-position (the bonding number of the hydrocarbon groups is 4), a combination of a 1-position, a 4-position, a 8-position and a 11-position (the bonding number of the hydrocarbon groups is 4), and a combination of a 2-position, a 3-position, a 9-position and a 10-position (the bonding number of the hydrocarbon groups is 4). A combination of a 6-position and a 13-position is most preferred; and a combination of a 5-position, a 7-position, a 12-position and a 14-position, a combination of a 1-position, a 4-position, a 8-position and a 11-position, and a combination of a 2-position, a 2-position, a 9-position and a 10-position are preferred in that order. A pentacene derivative tends to be oxidatively decomposed when dissolved in a solvent in air, but depending on the position of the hydrocarbon groups added thereto, the decomposition speed of the derivative may lower. This corresponds to an order having a higher spin density of $\pi$ electron cloud of pentacene.

Proc. Natl. Acad. Sci, U.S.A. 2014, 111, 7527-7530 shows that substitution of the hydrogen atoms contained

in pentacene with deuterium atoms increases the $^1$H spin polarization ratio to be attained by dynamic nuclear polarization.

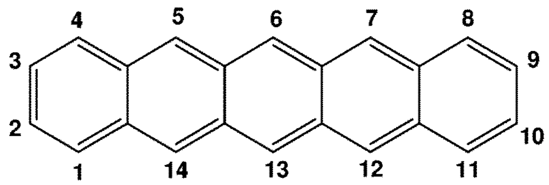

Examples of the pentacene derivative represented by the formula (A) and/or a salt thereof include the following compounds 1 to 13.

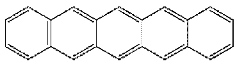

Compound 1

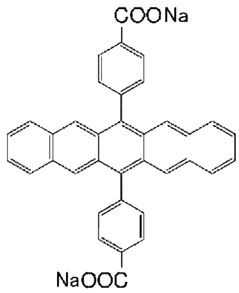

Compound 2

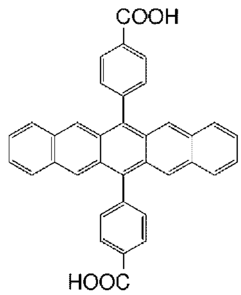

Compound 3

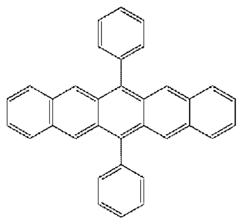

Compound 4

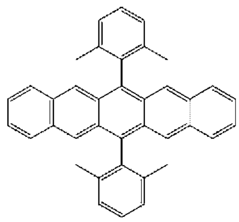

Compound 5

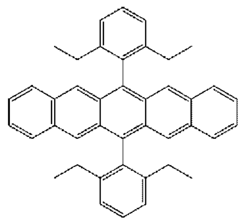

Compound 6

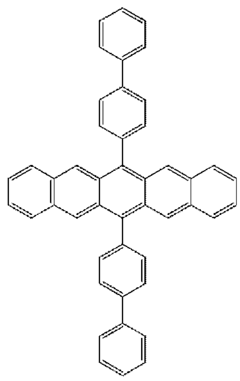

Compound 7

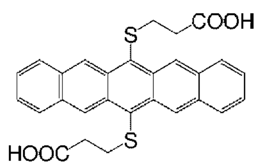

Compound 8

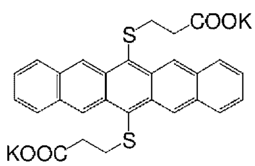

Compound 9

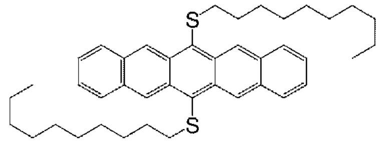

Compound 10

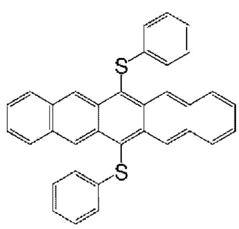

Compound 11

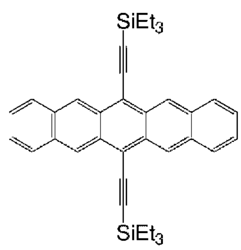

Compound 12

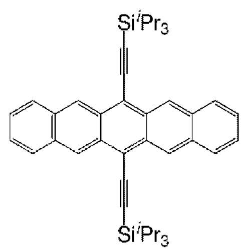

Compound 13

Among the above, the compound 1 (pentacene) and the compound 2 (PDBANa) were analyzed to measure the electron spin resonance spectrum thereof in Examples, and were confirmed to give an NMR signal.

Also J .Am. Chem. Soc. 2008, 130, 16274-16286 describes in detail the compounds 4, 5, 10, 11 and 13, for example, relating to the effect of the substituent thereof to be given to the HOMO-LUMO gap and the photooxidation resistance of pentacenes.

In addition, J. Mater. Chem. C, 2013, 1, 2193-2201 shows that pentacene derivatives such as the compounds 8 and 9 are soluble in water and are applicable to solar cells, but there is no report relating to use of such derivatives as a polarization source in dynamic nuclear polarization.

In the above-mentioned 13 kinds of compounds, a part or all of the hydrogen atoms (H) contained in the chemical formulae may be substituted with deuterium atoms (D).

Also a compound composed of a carbon atom, a hydrogen atom and a deuterium atom alone is preferably usable as a polarization source.

The content of the polarization source in a composition is preferably 10 μM to 100 mM, more preferably 100 μM to 20 mM, even more preferably 1 mM to 10 mM.

[Composite of Polarization Source and Host]

A polarization source may form a composite along with a host to be dispersed in an aqueous medium.

In this description, "host" means a substance capable of forming a composite along with a polarization source. A composite of a polarization source and a host may be in any form where a polarization source and a host are integrally combined, and the configuration thereof is not specifically limited. Namely, the "composite" as referred to herein widely includes a configuration of a molecule of a polarization source integrally combined with a host through inclusion, adsorption, adhesion or the like. Since a polarization source forms a composite along with a host, the dispersibility of the polarization source in an aqueous medium increases and the polarization source can be readily dispersed therein in a non-associated state. With that, triplet lifetime reduction owing to aggregation of a polarization source can be suppressed, and spin polarization of a triplet electron can be efficiently transitioned to a nucleus. Regarding the description of "dispersion in a non-associated state", reference may be made to the description of the column of "Dispersion State of Polarization Source" to be given hereinunder.

The host includes proteins such as albumin and globulin; oligosaccharides such as cyclodextrin; crystals of organic compounds such as p-terphenyl; cyclic compounds such as pillar arene, and calixarene; biopolymers such as gelatin and agarose; and crystalline porous polymers such as metal organic frameworks (MOF) and covalent-bonding organic skeleton frameworks (COF).

The configuration of the composite is not specifically limited, and may be amorphous or a shaped solid. In the case where the composite is a shaped solid, the shape is preferably granular, and is more preferably nanoparticles having a particle size of 800 nm or less. The particle size of the nanoparticles is preferably 500 nm or less, more preferably 200 nm or less, even more preferably 5 to 100 nm.

Here, the particle size of the particles can be measured through dynamic light scattering or with a scanning electron microscope or a transmission electron microscope.

Preferred examples of a granular composite include nanoparticles formed of a crystal or an organic compound, in which the crystal is doped with a polarization source.

The nanoparticles doped with a polarization source can be produced, for example, by melting a mixture of a polarization source and a crystalline compound, then cooling it to form a bulky crystal, and grinding the bulky crystal in a wet process. The nanoparticles produced here constitute aggregates of independent nanoparticles, and can be dispersed to a high extent in an aqueous medium. As opposed to this, in the case where particles are formed by a reprecipitation process or where bulk crystals are ground in a dry process, the particles may aggregate together to form a massive form and it is extremely difficult to obtain a composition having high dispersibility in the case.

Here, in grinding bulk crystals in a wet process, preferably, they are ground in the presence of a surfactant. In that manner, nanoparticles having higher dispersibility can be obtained.

In the case of using a composite of a polarization source and a host, the blend ratio of the polarization source and the host is preferably 1/50 to 1/10000, more preferably 1/100 to 1/1000, even more preferably 1/200 to 1/500.

The content of the composite in the composition is preferably 0.1% by weight to 20% by weight, more preferably 1% by weight to 15% by weight, even more preferably 5% by weight to 10% by weight.

[Aqueous Medium]

"Aqueous medium" in the present invention means a medium containing water. The proportion of water contained in the aqueous medium is preferably 90% or more.

The medium is preferably a liquid at room temperature (300 K), or a hydrogel.

The medium that is liquid at room temperature may be composed of water alone, or may be a mixed medium of water and any other medium. The other medium to be mixed with water is preferably a polar medium. Specific examples

of the polar medium include methanol, ethanol, isopropanol, acetonitrile, N,N-dimethylformamide, dimethyl sulfoxide, and glycerol. In the case where any other medium is mixed with water, the other medium may be one kind or may be two or more kinds.

A hydrogel is a solid medium formed of a network structure and a dispersion medium contained in the gaps of the network structure. A substance of forming the network structure includes hydrophilic polymers, low-molecular gelling agents and inorganic materials such as clay layers. The hydrophilic polymers include polysaccharides such as agarose, starch, glucomannan, carrageenan, pectine, methyl cellulose, curdlan, gellan gum, tamarind seed gum, and a mixture of xanthane gum and locust bean gum; and polyethylene glycol, polyvinyl alcohol, polyisopropylacrylamide, and polypeptide.

Regarding the description, the preferred range and the specific examples of the dispersion medium to be infiltrated into the hydrogel, reference may be made to the description relating to "the medium that is liquid at room temperature" mentioned above.

In the case where a hydrogel is used as the aqueous medium, the polarization source may be compounded with the hydrogel, or may be incorporated into the hydrogel by covalent bonding. Here, "compounding" in the case where a polarization source is compounded with a hydrogel widely includes a configuration of a molecule of a polarization source integrally compounded with the network structure to constitute a hydrogel through inclusion, adsorption, adhesion or the like. In that manner, when a polarization source is compounded with a hydrogel or incorporated into a gel by covalent bonding, association of polarization sources with each other is suppressed and shortening of triplet lifetime to be caused by aggregation of polarization sources can be thereby suppressed. As a result, spin polarization of triplet electrons can be efficiently transitioned to nuclei.

[Other Components]

The composition of the present invention can be composed of a polarization source and an aqueous medium alone, or can be composed of a polarization source, a host and an aqueous medium alone, or may contain any other component.

Hereinunder the other components that the composition can contain are described.

(Surfactant)

In the case where a polarization source constitutes a composite along with a host, and especially when the composite is particles having a particle size of 800 nm or less, the composite preferably contains a surfactant, and also preferably, a coating layer of a surfactant is formed on the surface thereof. With that, association of composites with each other can be suppressed, and composites can be uniformly dispersed in an aqueous medium. As a result, composites can be well irradiated with light and microwaves and spin polarization of triplet electrons can be efficiently transitioned to nuclei.

The surfactant may be any of a cationic surfactant, an anionic surfactant, an ampholytic surfactant and a nonionic surfactant, but is preferably a cationic surfactant. Preferred examples of the cationic surfactant include quaternary ammonium salt-type surfactants such as dialkyldimethylammonium salts, dimethyldialkylammonium halides, and dialkyldimethylammonium halides, and a specific example thereof is cetyltrimethylammonium bromide.

The amount of the surfactant to be added to the composition is preferably 1 to 0.001% by weight, more preferably 0.5 to 0.005% by weight, even more preferably 0.1 to 0.01% by weight.

[Dispersion State of Polarization Source]

In the composition of the present invention, preferably, a polarization source is dispersed in an aqueous medium in a non-associated state therein. Here, "a non-associated state" means that the molecules of a polarization source are not associated with each other but exist as spaced from each other, and this includes both a case where the molecules of a polarization source are dispersed in a monomolecular dispersion state (dispersed as individual molecules) and a case where a polarization source is dispersed and incorporated in a hydrogel by covalent bonding.

A polarization source in a non-associated state can be confirmed as follows. A fluorescence spectrum of a composition thereof is measured, and for example, in the case of a pentacene derivative, when a sharp fluorescence peak is observed in a wavelength range of 580 to 630 nm, the non-associated state of the composition is confirmed. Here, a sharp fluorescence peak means a fluorescence peak having a full width at half maximum of 70 nm or less. The full width at half maximum of the fluorescence peak is preferably 100 nm or less, more preferably 70 nm or less, even more preferably 50 nm or less.

[Mechanism and Method of Hypernuclear Polarization]

The composition of the present invention can be effectively used for hypernuclear polarization to increase the spin polarization ratio of a nucleus.

In the following, a mechanism of the composition of the present invention to increase the spin polarization ratio of a nucleus is described with reference to Fig. 1 that shows an example of using a molecule capable of taking an excited triplet state as a polarization source. It should be noted that the mechanism of the hypernuclear polarization with the composition of the present invention is not limitatively interpreted by the mechanism described below.

[1] Polarization Source Excitation Step

In this step, the composition is irradiated with excitation light to transition the polarization source therein of molecules capable of taking an excited triplet state into an excited triplet state.

When the composition is irradiated with excitation light, as shown in Fig. 1, the polarization source transitions from a ground singlet state So to an excited singlet state $S_1$, and further undergoes intersystem crossing from the excited singlet state $S_1$ to be in an excited triplet state $T_n$. Subsequently, the excited triplet state $T_n$ stepwise undergoes internal conversion into a lower-order excited triplet state, and finally into an excited triplet state $T_1$ at a lowest energy level. The number of the Zeeman level of the electron spin (triplet electron spin) in the excited triplet state $T_1$ corresponds to 3 of the magnetic quantum number m = -1, 0, +1, and is in an electron spin hyperpolarization state where the electron spins are distributed as greatly biased to a Zeeman level at m = 0 among these. On the other hand, the number of the Zeeman level of the nucleus of a host is, for example, for a proton $^1H^+$, 2 corresponding to the magnetic quantum number m = +1/2, -1/2. Among these, at the Zeeman level at m = +1/2, the nucleus spin population is slightly larger but the polarization ratio is $10^{-6}$ or so, and is in an extremely low nucleus spin polarization state.

[2] Hyperpolarization Step

In this step, the spin polarization of the triplet electrons formed in the composition is transitioned to nucleus for nuclear spin hyperpolarization.

Specifically, while an external magnetic field is applied thereto, the composition having a triplet electron spin formed therein is irradiated with electromagnetic waves for electron spin resonance. With that, owing to an integrated solid effect, the triplet electron spin polarization transitions to nuclei to induce nuclear spin hyperpolarization. At that time, in the composition of the present invention, the polarization source is highly dispersed in an aqueous medium, and therefore the triplet electron lifetime is long and transition of the spin polarization to nuclei continues for a long period of time, and spin polarization is accumulated in nuclei. Accordingly, an NMR signal having a high intensity can be obtained from a substance where the nuclear spin has been hyperpolarized (polarization target), and in NMR spectroscopy and MRI, a high measurement sensitivity can be realized in that condition.

The hyperpolarization condition is not specifically limited. For example, the strength of the external magnetic field can be appropriately selected from a range of 0.1 to 1 T, the frequency of electromagnetic waves can be from a range of 2 to 20 GHz, and the strength of the electromagnetic field can be from a range of 0.1 to 100 W.

Regarding the above-mentioned steps [1] and [2], the step [2] can be carried out after the step [1], or the step [1] and the step [2] can be carried out simultaneously. In the latter case, the composition is irradiated simultaneously with excitation light and electron spin-resonating electromagnetic waves while an external magnetic field is applied thereto.

The steps [1] and [2] can be carried out at room temperature but are preferably carried out at a temperature at which the aqueous medium used freezes. In that manner, a polarization source or a composite of a polarization source and a host can be kept in a highly dispersed state in a medium and accordingly, the triplet lifetime can be further longer and, in addition, polarization transition can be efficiently attained by the integrated solid effect. Moreover, the longitudinal relaxation time $T_1$ for nuclear spin such as $^1H$ of an aqueous medium can be prolonged and nuclear polarization can be accumulated more. Specifically, the temperature of the composition in the steps [1] and [2] is preferably 77 K to 300 K, more preferably 90 K to 150 K, even more preferably 100 K to 120 K.

The peak intensity of the NMR spectrum to be observed after hypernuclear polarization with the composition of the present invention is preferably 10 times or more relative to the peak intensity corresponding to the composition in a thermal equilibrium state, more preferably 100 times or more, even more preferably 1000 times or more.

The NMR signal can be detected in any known method of a continuous wave method or a pulse Fourier transform method. For example, NMR signal detection in a pulse Fourier transform method can be carried out using an apparatus equipped with an RF coil (probe) and an amplifier.

In the present invention, the nuclear spin of a target object to be measured is hyperpolarized using the composition of the present invention, and therefore an NMR signal can be detected at a high intensity from the target object.

Consequently, employing an NMR measurement method, a structure and physical properties of a compound can be analyzed according to an NMR spectroscopy method, and medical checkup of biological organs can be carried out by MRI at a high sensitivity.

[Polarization Target]

A polarization target to be subjected to hypernuclear polarization by the use of the composition of the present invention is not specifically limited, but preferably contains at least one compound selected from hydrocarbons and hydrocarbon derivatives in which at least one hydrogen atom is substituted with a substituent.

The hydrocarbons may be acyclic compounds (aliphatic compounds) or cyclic compounds, may be saturated hydrocarbons or unsaturated hydrocarbons, and may be low-molecular compounds or high-molecular compounds. The cyclic compounds may be alicyclic or aromatic compounds. The carbon number of the hydrocarbon is not specifically limited, and is generally within a range of 1 to 10. A part of the carbon atoms in the molecule of the hydrocarbon may be substituted with a hetero atom. The hetero atom is not specifically limited, including N, P, O and S.

In the hydrocarbon derivatives, the substituent is not specifically limited, but preferably, at least one substituent therein is a substituent containing an atom of which the spin quantum number I is any other than 0, more preferably a substituent containing $^{13}C$, $^{15}N$, $^{19}F$, $^{29}Si$ or $^{31}P$, even more preferably a fluorine atom.

Bio-related materials are especially preferred for the polarization target. Here, "bio-related materials" include substances constituting a living body, and derivatives of substances constituting a living body. Examples of the substances constituting a living body include biopolymers (nucleic acids, proteins, polysaccharides), and constituent elements thereof of nucleotides, nucleosides, peptides, amino acids and saccharides, as well as lipids, vitamins, and hormones. Specific examples of the bio-related materials to be the polarization target include molecules as $^{13}C$-labeled in the red-circled portion in Fig. 2(A) in Chem. Soc. Rev., 2014, 43, 1627-1659, which is hereby incorporated in a part of this description by reference. Introducing such a molecule as a probe into a living body, MRI-scopy can be carried out using the present invention.

<Composite>

Next the composite of the present invention is described.

The composite of the present invention contains a polarization source dispersed in a host, and is a composite having a particle size of 800 nm or less. The composite of the present invention can act to transition the electron spin polarization formed in the polarization source to a nucleus, and can be effectively used for dynamic nuclear polarization.

Regarding the description, preferred range and specific examples of "polarization source", reference may be made to the description of the section of [Polarization Source] given hereinabove, and regarding the description, preferred range and specific examples of "host", reference may be made to the corresponding description in the section of "Composite of Polarization Source and Host" given hereinabove.

"Particle size" as referred to in the present invention means a projected maximum diameter. "Particle size" can be measured through dynamic light scattering or with a scanning electron microscope.

The particle size of the composite is preferably 500 nm or less, more preferably 200 nm or less, even more preferably 5 to 100 nm.

Preferably, the composite is dispersed in an aqueous medium in use thereof. Regarding the description, preferred range and specific examples of "aqueous medium", reference may be made to the description of the section of "Aqueous Medium" given hereinabove.

Examples

The invention is described more specifically with reference to Synthesis Examples and Examples given below, in which the materials used, the details of the treatment and the treatment process may be appropriately modified or changed not overstepping the spirit and the scope of the invention. Accordingly, the invention should not be limitatively interpreted by the Examples mentioned below. For measurement of particle size distribution, a dynamic light scattering device (Nano-ZS ZEN3600, by Malvern Panalytical Ltd.) was used. For light absorption spectrometry, a spectrophotometer (V-670, V-770, by Japan Laser Corporation) was used. For fluorescence spectrometry, a multichannel spectrometer (MCPD-9800, by Otsuka Electronics Co., Ltd.) was used. For transient absorption measurement, a sample was irradiated with a 600-nm excitation light, and then the temporal change of the absorbance at 520 nm was measured. For NMR signal measurement, an NMR apparatus (JNM-ECA400, by JEOL Ltd.) was used. For time-resolved ESR spectrometry and dynamic nuclear polarization, a 532-nm pulse laser, an electromagnet, a microwave generator (HMC-T2220, by Analog Devices, Inc.) and an ESR detector were used as combined.

(Compounds used in Examples)

Polarization sources used in the present Examples are shown below.

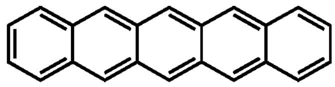

Pentacene

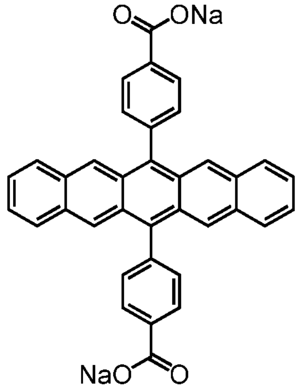

PDBANa

[1] Preparation and evaluation of aqueous dispersion of polarization source-containing composite

(Example 1) Production of aqueous dispersion of composite using pentacene as a polarization source and using p-terphenyl nanocrystals as a host

A mixture of pentacene and p-terphenyl was put into an ampule and sealed up in vacuum, and melted by heating them in an oil bath at higher than 220°C (melting point of p-terphenyl). Subsequently, the ampule was immersed in liquid nitrogen to rapidly cool the melt to give a bulky p-terphenyl doped with 0.5 mol% pentacene.

The bulky p-terphenyl crystal (300 mg), an aqueous solution of cetyltrimethylammonium bromide (1.4 mM, 30 mL) and alumina balls (diameter 5 mm) were put into a ceramic container, and ground at 280 rpm for 3 hours with a ball mill to give a milky white suspension. The suspension was centrifuged (6000 rpm, 5 minutes) to remove μm-order large granules, and a supernatant was thus collected. Subsequently, the supernatant was centrifuged (12000 rpm, 30 minutes) to precipitate nanoparticles. A part of the supernatant was removed, and the remaining supernatant and the precipitate (nanoparticles) were stirred to give an aqueous dispersion of nanoparticles (composite-containing aqueous dispersion 1). The content of the nanoparticles in the aqueous dispersion was 5.3% by weight as a solid weight.

Hereinunder the nanoparticles prepared herein are referred to as "pentacene-doped nanoparticles", and the bulky p-terphenyl crystal before grinding is referred to as "pentacene-doped bulky crystal".

A scanning electron microscope (SEM) picture of the pentacene-doped nanoparticles (magnification: 40000) is shown in Fig. 2; and a particle size distribution measured through dynamic light scattering (DLS) is shown in Fig. 3.

The particle size of the pentacene-doped nanoparticles is 92 ± 22 nm as an average particle size obtained from the SEM image, and is 151 ± 26 nm as an average particle size obtained through dynamic light scattering, and the data in the two measurement methods were well consistent. The zeta potential of the pentacene-doped nanoparticles was +58 mV, and was a large positive value. This is considered to be because of the surface coating layer of the cationic surfactant, cetyltrimethylammonium bromide. The pentacene-doped nanoparticles were given good colloid stability by the charged surface, and the nanoparticles dispersed stably over 24 hours.

The pentacene-doped nanoparticles and the pentacene-doped bulk crystal were analyzed in point of the powdery X-ray diffraction pattern, the photoabsorption spectrum, the fluorescence lifetime and the transient absorption curve, and the found data well coincided between the two. Here, regarding the pentacene-doped bulk crystal, it is known that pentacene exists in the crystal in a molecular dispersion state, and the finding that the pentacene-doped nanoparticles had the same properties as those of the pentacene-doped bulk crystal means that pentacene exists in a molecular dispersion state also in the pentacene-doped nanoparticles.

The prepared aqueous dispersion of pentacene-doped nanoparticles was photoexcited by irradiation with a 532-nm pulse laser, and further irradiated with microwaves along with magnetic field sweeping to repeat dynamic nuclear polarization of transitioning the triplet electron spin polarization formed in pentacene to $^1$H, for a predetermined period of time. The $^1$H NMR spectrum before dynamic nuclear polarization and the $^1$H NMR spectrum after dynamic nuclear polarization repeated for a predetermined period of time are shown in Fig. 4. Here, the measurement frequency for the $^1$H NMR spectrum was at intervals of 15 seconds within 60 seconds. The $^1$H NMR spectrum after dynamic nuclear polarization repeated for 60 seconds is shown in Fig. 5(a); and an enlarged view of the $^1$H NMR spectrum in a range of

-20 to -15 kHz is shown in Fig. 5(b). Figs. 5(a) and 5(b) show both a simulation curve obtained by curve fitting of the actually-measured $^1$H NMR spectrum, and two spectral components divided from the spectrum. Among the two spectral components, a sharp peak is derived from $^1$H of water, and a relatively broad peak is derived from $^1$H of pentacene-doped nanoparticles.

As shown in Fig. 4, the $^1$H NMR spectrum before dynamic nuclear polarization gave a sharp peak alone derived from water. On the other hand, the $^1$H NMR spectrum after dynamic nuclear polarization gave a relatively broad peak derived from pentacene-doped nanoparticles, in which the peak tended to be sharper with increase in the repetition time for dynamic nuclear polarization. From this, it is confirmed that by dynamic nuclear polarization of pentacene-doped nanoparticles in water, the nuclear spin polarization increases.

The results suggest the possibility that water serving as a medium has an ability of transitioning spin polarization, and it is known that a constitution of dispersing a polarization source in a water medium can be an evolutionary approach to a technique of transitioning spin polarization from a polarization source to water.

A $^1$H spin polarization buildup curve of an aqueous dispersion of pentacene-doped nanoparticles and a pentacene-doped bulk crystal, and a fitting curve of the buildup curve are shown in Fig. 6. The horizontal axis of Fig. 6 shows a dynamic nuclear polarization repetition time. The vertical axis shows an enhancement factor, which was obtained by substituting the found $^1$H NMR signal (signal voltage) data for $E_{DNP}$ in the following formula (1). Here, as reference data, $^1$H NMR signal data of pentacene-doped bulk crystal in a thermal equilibrium state in a magnetic field at 0.676 T at 300 K were used.

$$\varepsilon = \frac{N_{ref}}{N_{DNP}} \frac{T_{DNP}}{T_{ref}} \frac{g_{ref}}{g_{DNP}} \frac{E_{DNP}}{E_{ref}} \qquad \cdots (1)$$

In the formula (1), $N_{DNP}$ and $N_{ref}$ each represent a number of $^1$H spins, $T_{DNP}$ and $T_{ref}$ reach represent a temperature, $g_{DNP}$ and $g_{ref}$ each represent a receiver gain, $E_{DNP}$ and $E_{ref}$ each represent a signal voltage. Among these, symbols with a subscript "DNP" are parameters for the compounds used as a polarization source, and symbols with a subscript "ref" are parameters for pentacene-doped bulk crystals.

(Comparative Example 1) Preparation of aqueous dispersion of PDBANa (aqueous dispersion of polarization source not in a molecular dispersion state)

PDBANa was dispersed in water at a concentration of 0.1 mM to prepare an aqueous dispersion thereof.

The aqueous dispersion of PDBANa and a methanol solution of PDBANa in a molecular dispersion state (reference sample) were analyzed to measure the fluorescence spectrum, which confirmed that the fluorescence peak from the aqueous dispersion of PDBANa has an obviously broad form as compared with that from the methanol solution thereof. From this, it is known that PDBANa slightly aggregates in water and could not be in a complete molecular dispersion state. In addition, after degassed, the aqueous dispersion and the methanol solution of PDBANa each were irradiated with excitation light, and subjected to transient absorptiometry as an index of a triplet lifetime. The attenuation rate of the aqueous dispersion was higher than that of the methanol solution. This indicates that since PDBANa is not in a molecular dispersion state in the aqueous dispersion, its triplet lifetime is short.

(Example 2) Production of aqueous dispersion of composite using PDBANa as a polarization source and using bovine serum albumin (BSA) as a host

An aqueous 0.1% BSA solution was added to a powder of PDBANa and then left in a dark place for 10 minutes to prepare an aqueous dispersion containing a composite of PDBANa and BSA (composite-containing aqueous dispersion 2).

Thus produced, the aqueous dispersion of composite was analyzed to measure the fluorescence spectrum, in which the fluorescence peak shifted toward a short wavelength side and the peak shape was sharp as compared with that in the spectrum from the aqueous dispersion of PDBANa alone produced in Reference Example 1. From this, it is known that the dispersibility of PDBANa in the composite with BSA improved. The produced aqueous dispersion of composite was analyzed to measure the circular dichroism (CD) spectrum, which showed a significant CD change not seen in the CD spectrum of aqueous dispersion of PDBANa alone or aqueous solution of bovine serum albumin alone. From this, it is confirmed that the PDBANa molecules were incorporated in BSA with chirality.

Further, the degassed aqueous dispersion of composite was irradiated with excitation light, and then subjected to transient absorption measurement. The attenuation rate of the composite dispersion was lower than that of the aqueous dispersion of PDBANa alone, and the triplet lifetime thereof was 65 μs and was on the same level as that a methanol solution of PDBANa in a molecular dispersion state (66 μs). From this, it is known that in the aqueous dispersion of

composite, PDBANa was incorporated in BSA to be in a molecular dispersion state, and can therefore realize a long triplet lifetime.

(Example 3) Production of aqueous dispersion of composite using PDBANa as a polarization source and using β-cyclodextrin as a host

β-cyclodextrin was added to an aqueous dispersion of PDBANa to prepare an aqueous dispersion containing a composite of PDBANa and β-cyclodextrin (composite-containing aqueous dispersion 3). Here, the concentration ratio of PDBANa to β-cyclodextrin (PDBANa/β-cyclodextrin) in the aqueous dispersion was varied according to the measurement to be carried out. The employed concentration ratio is shown in the subsequent parenthesis before each aqueous dispersion.

Thus produced, the aqueous dispersion of composite (concentration ratio, 0.1 mM/1 mM) was analyzed to measure the fluorescence spectrum, in which the fluorescence peak shifted toward a short wavelength side and the peak shape was sharp as compared with that in the spectrum from the aqueous dispersion of PDBANa alone produced in Reference Example 1. From this, it is known that the dispersibility of PDBANa in the composite with β-cyclodextrin improved. In addition, a heavy aqueous solution of PDBANa/β-cyclodextrin = 1 mM/5 mM was prepared, and its $^1$H NMR spectrum was measured. In this, a peak position derived from β-cyclodextrin significantly changed relative to that from a heavy aqueous solution of β-cyclodextrin alone. Such a peak position change shows a possibility that β-cyclodextrin could form an inclusion with PDBANa, suggesting that this contributes toward molecular dispersion of PDBANa.

Further, the degassed aqueous dispersion of composite (concentration ratio, 0.5 mM/5 mM) was irradiated with 600-nm excitation light, and then subjected to transient absorption measurement at 520 nm to measure the triplet lifetime thereof. The triplet lifetime was 76 μs, and was longer than the triplet lifetime (66 μs) of a methanol solution of PDBANa (0.1 mM) in a molecular dispersion state. The reason why such a long triplet lifetime was attained is considered to be because, in the aqueous dispersion of composite, PDBANa was in a molecular dispersion state and, in addition, the side chain mobility of β-cyclodextrin therein was limited.

<u>[2] Preparation and evaluation of gel containing a polarization source-containing composite</u>

(Example 4) Production of composite-containing gel 1 using PDBANa as a polarization source, using β-cyclodextrin as a host and using agarose hydrogel as a medium

An aqueous dispersion of 4% by weight agarose was fully dissolved by heating at 95°C, then mixed with an aqueous dispersion containing PDBANa (3 mM) and β-cyclodextrin (10 mM) in a ratio by volume of 1/1, and solidified by cooling in a refrigerator to give a gel (a composite-containing gel 1 using agarose gel as a medium).

The resultant composite-containing gel 1 exhibited a pink color similar to the aqueous dispersion of a composite of PDBANa and β-cyclodextrin produced in Example 3 (composite-containing aqueous dispersion 3). Its fluorescence spectrum was measured, which gave a sharp fluorescence peak at the same position as that from the composite-containing aqueous dispersion 3. This confirms that, in the composite-containing gel 1, PDBANa and β-cyclodextrin form a composite therein, and PDBANa is in a molecular dispersion state.

(Example 5) Production of composite-containing gel 2 using PDBANa as a polarization source, using β-cyclodextrin as a host and using polyethylene glycol hydrogel (tetra-PEG) as a medium

According to the reaction scheme shown in Fig. 7, a composite-containing gel 2 was produced in which a composite of PDBANa and β-cyclodextrin is incorporated in a polyethylene glycol hydrogel by covalent bonding.

Specifically, first, a methanol solution of 1.5 mM PDBANa (200 μL) was added to a mixture of 4arm-PEG5K-NH2 (5 mg) and 4-(4,6-dimethoxy-1,3,5-triazin-2-yl)-4-methylmorpholynium chloride (DMT-MM, 1 mg), and left in a dark place for 3 hours. The solvent was evaporated away from the mixture, then 4arm-PEG5K-NH2 (5 mg) and DMT-MM ( 1 mg) were additionally added thereto, and further an aqueous solution of 50 mM sodium terephthalate (74 μL) and methanol (126 μL) were added, and then left in a dark place for 6 hours. The resultant pink gel was immersed in an aqueous solution of 10 mM β-cyclodextrin, and the solvent and the monomer were washed away to give a blue gel (a composite-containing gel 2 using tetra-PEG as a medium).

The resultant composite-containing gel 2 was analyzed to measure the fluorescent spectrum, in which a sharp fluorescence peak was detected at the same position as that of the composite-containing aqueous dispersion 3 of Example 3. This confirms that, also in this composite-containing gel 2, aggregation by association of PDBANa did not occur, and PDBANa existed in a molecular dispersion state.

(Comparative Example 2) Production of gelatin gel containing a composite of PDBANa and β-cyclodextrin (comparative gel 1)

An aqueous dispersion of 4% by weight gelatin was fully dissolved by heating at 55°C, then mixed with an aqueous dispersion containing PDBANa (3 mM) and β-cyclodextrin (10 mM) in a ratio by volume of 1/1, and solidified by cooling in a refrigerator to give a gel (comparative gel 1).

The resultant gel was analyzed to measure the fluorescence spectrum, in which the fluorescence peak greatly shifted toward a long wavelength side as compared with the composite-containing aqueous dispersion 3 of Example 3 and the composite-containing gel 1 of Example 4. This indicates that, in the gel, PDBANa was in an aggregated state and the dispersibility thereof is poor. The aggregation is considered to be caused by interaction between the ionic site of the gelatin gel and PDBANa.

The composite-containing aqueous dispersion 3 (1.5 mM/5 mM) produced in Example 3, the composite-containing gel 1 produced in Example 4, the composite-containing gel 2 produced in Example 5 and the comparative gel 1 produced in Comparative Example 2 each were individually frozen at 120 K, and then photoexcited by irradiation with a 532-nm pulse laser in a magnetostatic field, and thereafter the ESR (electron spin resonance) spectrum thereof was measured by irradiation with microwaves with sweeping the magnetic field. A largest value of the ESR signal was seen at around 350 to 355 mT, and the temporal change of the ESR peak is shown in Fig. 8.

As shown in Fig. 8, the composite-containing aqueous dispersion 3, the composite-containing gel 1 using agarose gel, and the composite-containing gel 2 using tetra-PEG gave ESR peaks, and it is known that the ESR peaks attenuated with time. On the other hand, the comparative gel 1 using the comparative gel 1 did not give an ESR peak, which indicates that electron spin polarization did not almost occur in the comparative gel. From this, it is known that for providing electron spin polarization, it is necessary that the molecules of the polarization source do not aggregate and disperse to a high extent. The composite-containing gels 1 and 2 gave larger ESR signals than the aqueous dispersion of composite, which indicates that using a hydrogel as a medium of a polarization source is advantageous for enhancing electron spin polarization. This is considered to be because the dispersibility of the polarization source improved more in the frozen agarose gel or tetra-PEG gel than in mere ice.

The ESR spectrum of the composite-containing gel 1 was analyzed. In this, a peak in the negative direction was detected at around 310 mT, and a peak in the positive direction was at around 350 mT. These peaks are derived from transition between sub-levels in an excited triplet state (transition from m = 0 to m = -1, transition from m = 0 to m = +1). From this, it is confirmed that by the microwave irradiation and the magnetic field sweeping as above, triplet state spin polarization formed in PDBANa in the hydrogel.

Further, the composite-containing gel 1 using agarose gel was photoexcited by irradiation with a 532-nm pulse laser at 100 K, and then subjected to dynamic nuclear polarization with a microwave pulse for a predetermined period of time to thereby transition the triplet electron spin polarization formed in PDBANa to $^1H$. Fig. 9 shows a $^1H$ NMR spectrum after 3 minutes and 90 minutes of dynamic nuclear polarization, and Fig. 10 shows pulse irradiation time dependency of the NMR signal integrated value.

As shown in Fig. 9 and Fig. 10, the NMR signal tended to increase with increase in the pulse irradiation time. From this, it is confirmed that in the composite-containing gel 1, PDBANa surely attains spin polarization transition from triplet electron to $^1H$. In addition, since a time on a few minutes scale is taken for signal sensitization, it is known that the longitudinal relaxation time that is to be an index of time for retaining polarization is extremely long.

Also the composite-containing gel 2 using tetra-PEG was subjected to the same dynamic nuclear polarization. The case where PDBANa is a monomer also showed increase in the NMR signal, and was found to have a long relaxation time and maintain crystallinity.

Industrial Applicability

The present invention enables hypernuclear polarization in an aqueous medium. The composition of the present invention uses an aqueous medium, and is readily applicable to living bodies and bio-related materials. Consequently, the industrial applicability of the present invention is great.

## Claims

1. A composition of a polarization source dispersed in an aqueous medium.

2. The composition according to claim 1, wherein the polarization source is dispersed in an aqueous medium in a non-associated state therein.

3. The composition according to claim 1 or 2, wherein a composite of the polarization source and a host is dispersed in an aqueous medium.

4. The composition according to claim 3, wherein the composite is particles having a particle size of 800 nm or less.

5. The composition according to claim 3 or 4, wherein the composite contains a surfactant.

6. The composition according to claim 3 or 4, wherein the host is a protein.

7. The composition according to claim 6, wherein the host is an albumin.

8. The composition according to claim 3 or 4, wherein the host is an oligosaccharide.

9. The composition according to claim 8, wherein the oligosaccharide is a cyclodextrin.

10. The composition according to any one of claims 1 to 9, wherein the aqueous medium is water.

11. The composition according to any one of claims 1 to 9, wherein the aqueous medium is a hydrogel.

12. The composition according to claim 11, wherein the polarization source forms a composite with the hydrogel.

13. The composition according to claim 11, wherein the polarization source is incorporated in the hydrogel by covalent bonding.

14. A composite of a polarization source dispersed in a host and having a particle size of 800 nm or less.

15. The composite according to claim 14, having a surfactant.

16. The composite according to claim 14 or 15, wherein the host is a protein.

17. The composite according to claim 16, wherein the host is an albumin.

18. The composite according to claim 14 or 15, wherein the host is an oligosaccharide.

19. The composite according to claim 18, wherein the oligosaccharide is a cyclodextrin.

Fig. 1

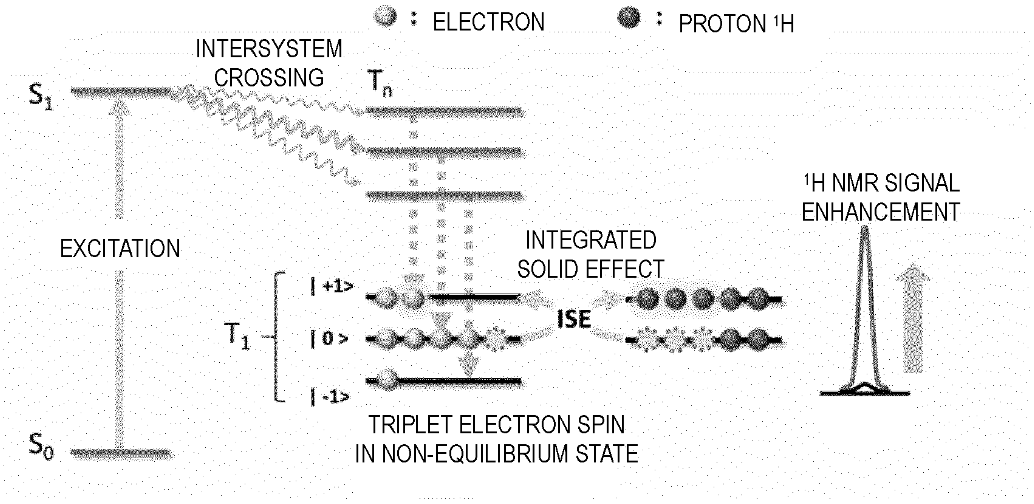


Fig. 2

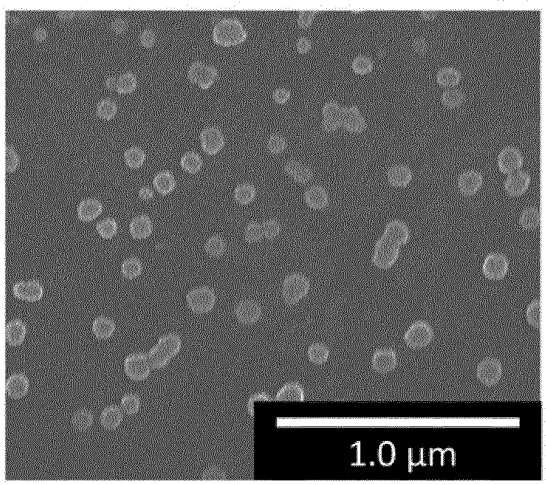


Fig. 3

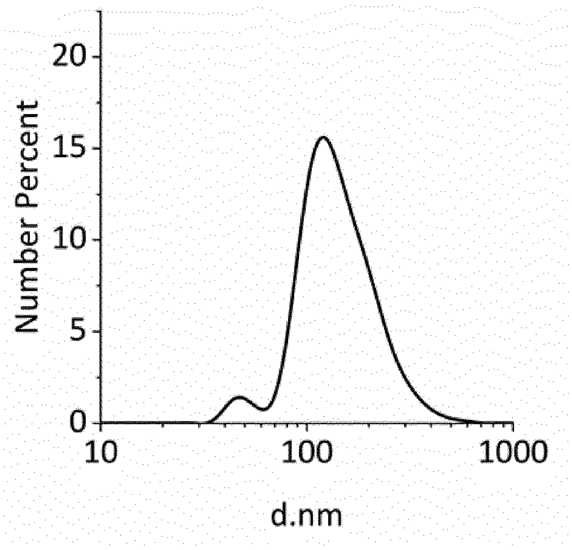

Fig. 4

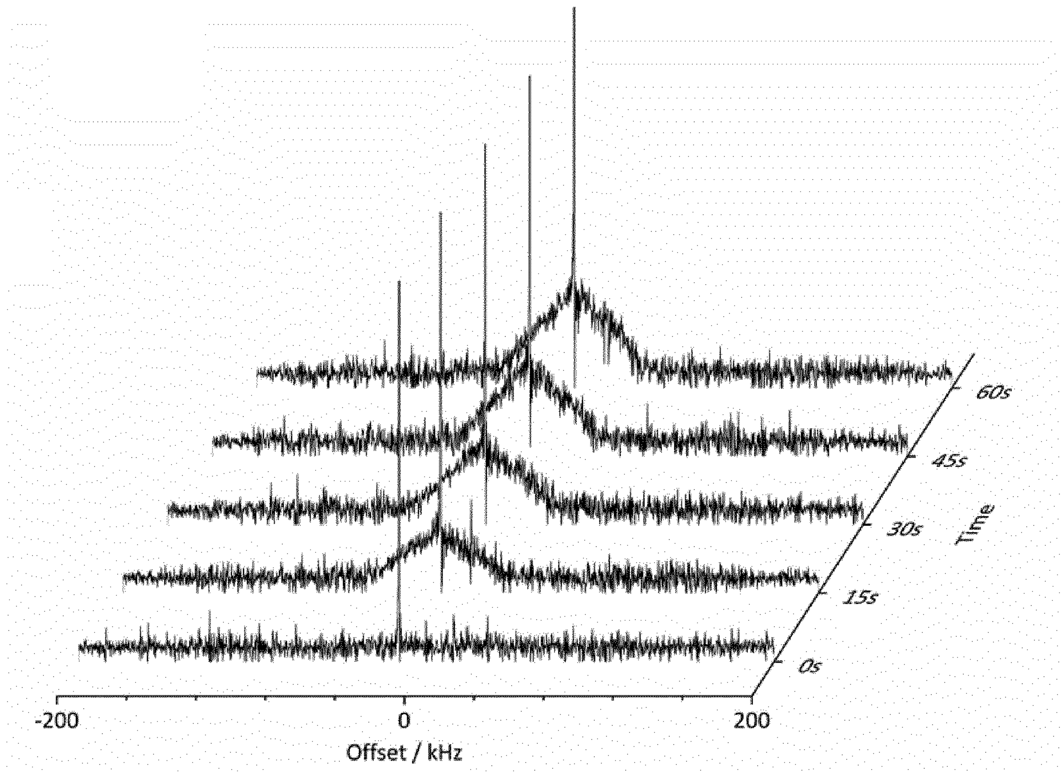
60s
45s
30s
15s
0s
Time
-200
0
200
Offset / kHz

Fig. 5

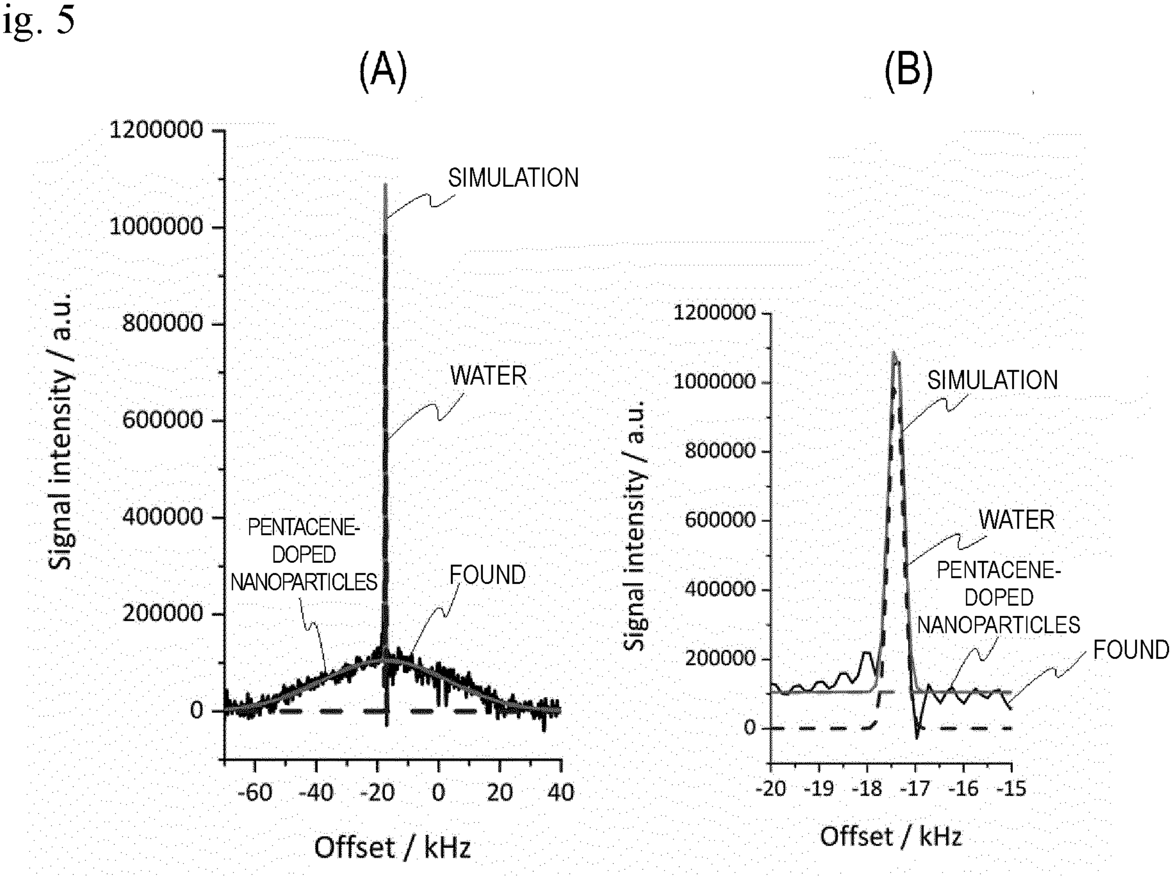
(A)
(B)
1200000
1000000
800000
600000
400000
200000
0
Signal intensity / a.u.
SIMULATION
WATER
PENTACENE-DOPED NANOPARTICLES
FOUND
-60
-40
-20
0
20
40
Offset / kHz
-20
-19
-18
-17
-16
-15

Fig. 6

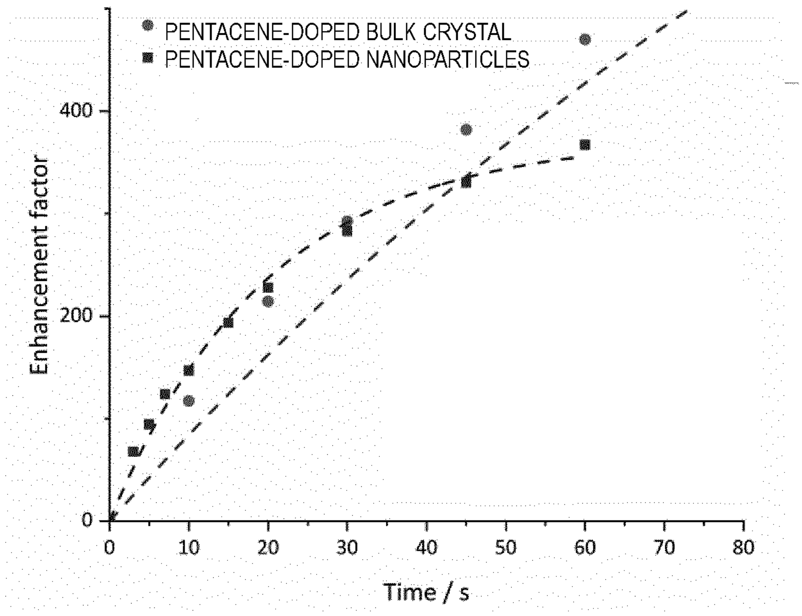
PENTACENE-DOPED BULK CRYSTAL
PENTACENE-DOPED NANOPARTICLES
Enhancement factor
0
200
400
0
10
20
30
40
50
60
70
80
Time / s

Fig. 7

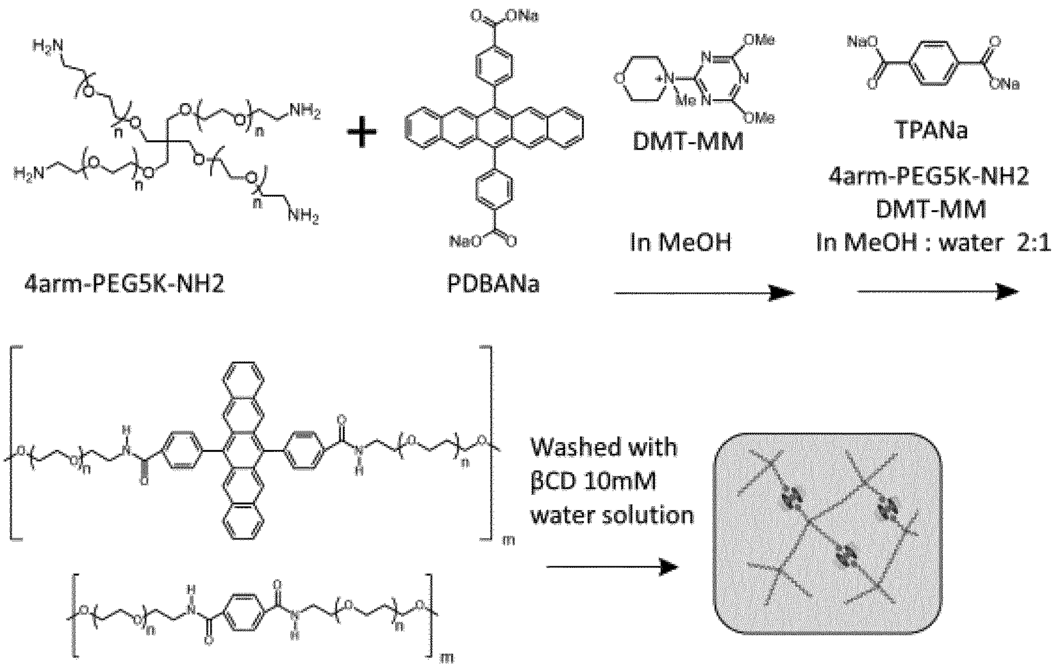
4arm-PEG5K-NH2
PDBANa
DMT-MM
In MeOH
TPANa
4arm-PEG5K-NH2
DMT-MM
In MeOH : water 2:1
Washed with
βCD 10mM
water solution

Fig. 8

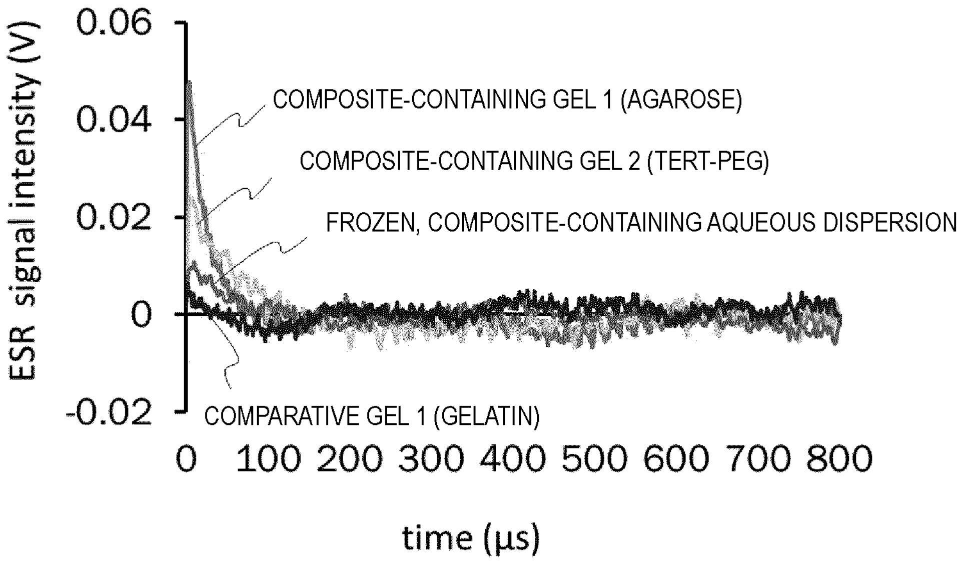
0.06
0.04
0.02
0
-0.02
ESR signal intensity (V)
COMPOSITE-CONTAINING GEL 1 (AGAROSE)
COMPOSITE-CONTAINING GEL 2 (TERT-PEG)
FROZEN, COMPOSITE-CONTAINING AQUEOUS DISPERSION
COMPARATIVE GEL 1 (GELATIN)
0 100 200 300 400 500 600 700 800
time (μs)

Fig. 9

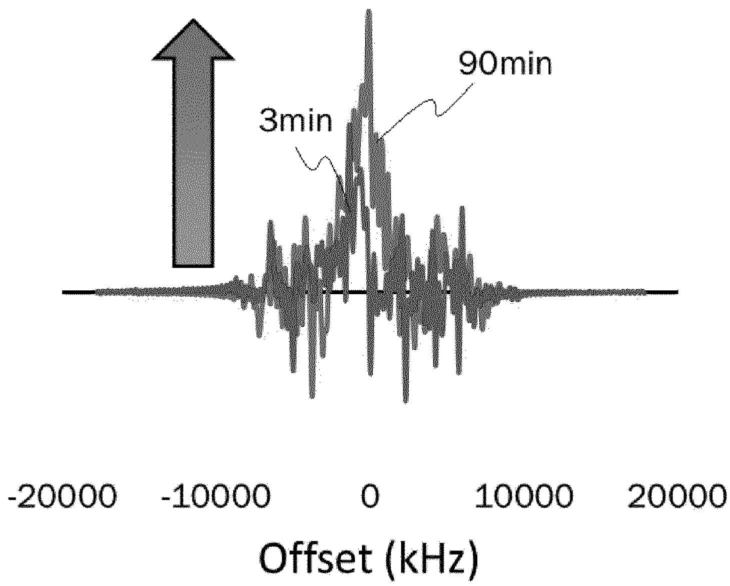
90min
3min
-20000 -10000 0 10000 20000
Offset (kHz)

Fig. 10

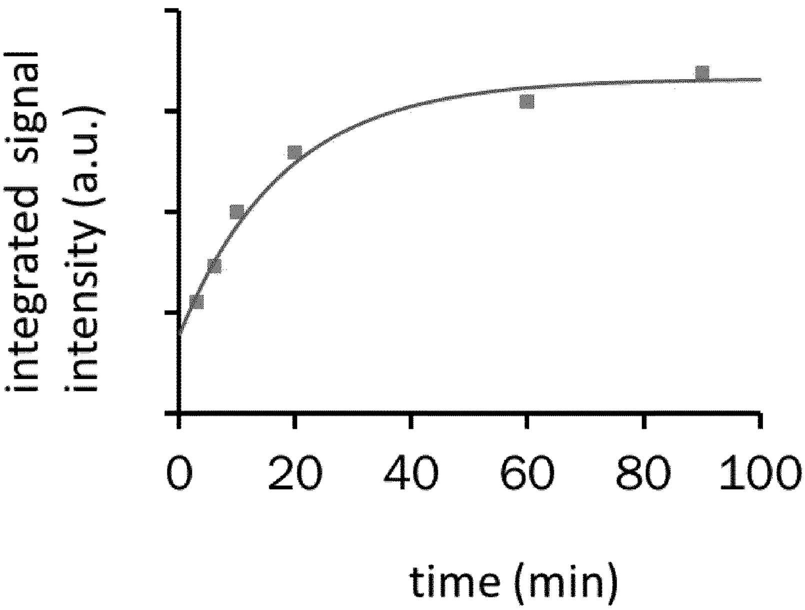

**INTERNATIONAL SEARCH REPORT**

International application No. PCT/JP2020/008481

A. CLASSIFICATION OF SUBJECT MATTER
C08L 5/16(2006.01)i; C08L 89/00(2006.01)i; A61K 49/08(2006.01)i; A61K 49/12(2006.01)i; A61K 49/14(2006.01)i; G01N 24/00(2006.01)i
FI: G01N24/00 100B; A61K49/08; A61K49/12; A61K49/14; C08L5/16; C08L89/00
According to International Patent Classification (IPC) or to both national classification and IPC

B. FIELDS SEARCHED
Minimum documentation searched (classification system followed by classification symbols)
G01N24/00-24/14; A61B5/055; G01R33/20-33/64; A61K49/06-49/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2017-15443 A (RIKEN, INSTITUTE OF PHYSICAL AND CHEMICAL RESEARCH) 19.01.2017 (2017-01-19) paragraphs [0015]-[0021] | 1, 10 |
| Y | paragraphs [0015]-[0021] | 2-9, 11-19 |
| Y | FUJIWARA, Saiya, "Dynamic Nuclear Polarization of Metal-Organic Frameworks Using Photoexcited Triplet Electrons", J. Am. Chem. Soc., 07 November 2018, vol. 140, pp. 15606-15610 column "abstract" | 2-9, 11-19 |
| Y | ナノ多孔性材料を室温で高核偏極化することに世界で初めて成功～生体分子の高感度 MRI 観測へ新たな道～, 九州大学プレスリリース［オンライン], 2018.11.08, http://kyushu-u.ac.jp/f/34490/18_11_08_1. pdf, [retrieval date 13 May 2020] entire text, non-official translation ("World's first success high nucleation and polarization of nanoporous materials at room temperature - New way through high sensitive MRI observation of biomolecules -", KYUSHU UNIVERSITY press release [online]) | 2-9, 11-19 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
13 March 2020 (13.03.2020)

Date of mailing of the international search report
02 June 2020 (02.06.2020)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.
PCT/JP2020/008481

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2015-532661 A (UNIVERSITE CLAUDE BERNARD LYON I) 12.11.2015 (2015-11-12) claims 1, 7, 10, 34 | 5, 11-13, 15 |
| Y | JP 11-501839 A (IMARX PHARMACEUTICAL CORP.) 16.02.1999 (1999-02-16) page 12, lower right column, lines 23-28, page 16, lines 5-7, page 17, line 13-24, page 24, lines 2-6 | 6-7, 11-13, 16-17 |
| Y | JP 2001-503646 A (LAWRENCE BERKELEY NATIONAL LABORATORY) 21.03.2001 (2001-03-21) page 52, lines 8-28 | 8-9, 11-13, 18-19 |
| A | US 2005/0107696 A1 (GRIFFIN, Robert G.) 19.05.2005 (2005-05-19) entire text, all drawings | 1-19 |

Form PCT/ISA/210 (continuation of second sheet) (January 2015)

**INTERNATIONAL SEARCH REPORT**

International application No.
PCT/JP2020/008481

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2020/008481

<Continuation of Box No. III>

Document 1: JP 2017-15443 A (RIKEN, INSTITUTE OF PHYSICAL AND CHEMICAL RESEARCH) 19.01.2017 (2017-01-19) paragraphs [0015]-[0021] paragraphs [0015]-[0021] & US 2018/0188338 A1 paragraphs [0064]-[0082] & WO 2017/002761 A1 & EP 3315987 A1

(Invention 1) Claims 1-13

Claims 1-13 pertain to a composition in which a polarization source is dispersed in an aqueous medium, and are classified as invention 1.

(Invention 2) Claims 14-19

Claims 14-19 share a common technical feature of including a "polarization source" with claim 1 classified as invention 1. However, this technical feature does not make a contribution over the prior art in light of the disclosure of document 1, and is thus not considered to be a special technical feature. Furthermore, these inventions share no other identical or corresponding special technical features.

Furthermore, claims 14-19 are not dependent on claim 1. Furthermore, claims 14-19 are not substantially identical or equivalent to any of the claims classified as invention 1.

Therefore, claims 14-19 cannot be classified as invention 1.

Claims 14-19 have a special technical feature of a composite in which a polarization source is dispersed in a host and which has a particle diameter of 800 nm or less. Thus, claims 14-19 are classified as invention 2.

Form PCT/ISA/210 (extra sheet) (January 2015)

**INTERNATIONAL SEARCH REPORT**
Information on patent family members

International application No.
PCT/JP2020/008481

| Patent Documents referred in the Report | Publication Date | Patent Family | Publication Date |
|---|---|---|---|
| JP 2017-15443 A | 19 Jan. 2017 | US 2018/0188338 A1 paragraphs [0064]-[0082]<br>WO 2017/002761 A1<br>EP 3315987 A1 | |
| JP 2015-532661 A | 12 Nov. 2015 | WO 2014/023659 A1 claims 1, 7, 10, 34<br>EP 2880458 A1<br>CN 104520727 A<br>US 2015/0219734 A1 | |
| JP 11-501839 A | 16 Feb. 1999 | EP 852477 A1<br>WO 1996/028090 A1 page 9, lines 22-29, page 13, lines 20-23, page 14, lines 36-14, page 22, lines 26-31<br>US 6088613 A | |
| JP 2001-503646 A | 21 Mar. 2001 | WO 1997/037239 A1 page 37, lines 1-18<br>EP 890114 A<br>US 2002/0094317 A1 | |
| US 2005/0107696 A1 | 19 May 2005 | WO 2005/024440 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *PNAS,* 2014, vol. 111, 7529
- *Angew. Chem. Int. Ed.,* 2013, vol. 52, 13307
- *Proc. Natl. Acad. Sci, U.S.A.,* 2014, vol. 111, 7527-7530
- **ALSO J .** *Am. Chem. Soc.,* 2008, vol. 130, 16274-16286
- *J. Mater. Chem. C,* 2013, vol. 1, 2193-2201
- *Chem. Soc. Rev.,* 2014, vol. 43, 1627-1659